# EUROPÄISCHE PATENTSCHRIFT

(11) **EP 4 048 203 B1**
(45) Veröffentlichungstag und Bekanntmachung des Hinweises auf die Patenterteilung: **29.11.2023**
(21) Anmeldenummer: 20799624.0
(22) Anmeldetag: 15.10.2020
(51) Int. Cl.: A61F 2/16

(54) **INJEKTORANORDNUNG FÜR EIN EINFÜHREN EINER INTRAOKULARLINSE**
INJECTOR ASSEMBLY FOR INSERTING AN INTRAOCULAR LENS
ENSEMBLE INJECTEUR POUR L'INSERTION D'UNE LENTILLE INTRAOCULAIRE

(30) Priorität: 21.10.2019 DE 102019128372
(43) Veröffentlichungstag der Anmeldung: 31.08.2022
(73) Patentinhaber: Carl Zeiss Meditec AG, 07745 Jena (DE)
(72) Erfinder: RATHERT, Brian, 45659 Recklinghausen (DE); PANKIN, Dmitry, 12623 Berlin (DE); RAQUIN, Vincent, 17000 La Rochelle (FR)
(74) Vertreter: PATERIS Patentanwälte PartmbB
(86) Internationale Anmeldenummer: PCT/EP2020/079016
(87) Internationale Veröffentlichungsnummer: WO 2021/078621

(56) Entgegenhaltungen:
- EP-A1- 2 101 685
- EP-B1- 2 101 685
- GB-A- 2 493 017
- US-A1- 2002 082 609
- US-A1- 2008 147 080

## Beschreibung

Die Erfindung betrifft einen Injektor für ein Einführen einer Intraokularlinse in den Kapselsack eines Auges.

Eine hydrophile Intraokularlinse ist für ihre Lagerstabilität in einem wässrigen Medium zu lagern. Ein Injektor zum Einführen der Intraokularlinse in den Kapselsack weist eine Beschichtung auf, die ein Gleiten der Intraokularlinse in dem Injektor erleichtert. Dies ist besonders relevant an Stellen des Injektors, an denen die Intraokularlinse besonders stark komprimiert ist. Weil sich die Beschichtung bei Kontakt mit Wasser auflöst, ist der Injektor im Gegensatz zu der Intraokularlinse trocken zu lagern. Die Intraokularlinse wird herkömmlich zusammen mit einer Aufbewahrungsflüssigkeit in einer Kammer gelagert, die in den Injektor eingesetzt wird. Vor einem Betrieb des Injektors ist die Kammer mittels einer Dichtung gegenüber den Injektor abzudichten. In dem Betrieb des Injektors ist die Dichtung zu öffnen, beispielsweise indem die Dichtung mittels eines Kolbens des Injektors durchstoßen wird. Wenn sich die Intraokularlinse beim Durchstoßen der Dichtung zwischen dem Kolben und der Dichtung befindet, oder wenn die durchstoßene Dichtung mittels des Kolbens auf die Intraokularlinse gedrückt wird oder wenn die Intraokularlinse die durchstoßene Dichtung passiert, kann die Intraokularlinse beschädigt werden, was einen großen Nachteil darstellt.

GB 2 493 017 A offenbart eine Kassette für eine Intraokularlinse und eine Injektionsvorrichtung für eine Intraokularlinse. US 2008/147080 A1 offenbart ein Intraokularlinseninjektionskit. EP 2 101 685 A1 offenbart Intraokularlinseninjektionskit. US 2002/082609 A1 offenbart ein Verpackungssystem für eine Intraokularlinse.

Aufgabe der Erfindung ist es, eine Injektoranordnung zu schaffen, mit der ein Risiko einer Beschädigung der Intraokularlinse gering ist.

Die erfindungsgemäße Injektoranordnung weist ein Einsetzbauteil und einen Injektor auf, der einen ersten Anschlag und eine Aufnahme aufweist, die eine erste Wand und eine zweite Wand aufweist, die beabstandet zueinander angeordnet sind, und eingerichtet ist, zwischen der ersten Wand und der zweiten Wand das Einsetzbauteil gleitend aufzunehmen, das eine Aufbewahrungsflüssigkeit und eine Intraokularlinse, die in einem Inneren des Einsetzbauteils umgeben von der Aufbewahrungsflüssigkeit angeordnet ist, sowie eine erste Dichtung aufweist, die außen an dem Einsetzbauteil angeordnet ist und das Innere des Einsetzbauteils abdichtet, wobei das Einsetzbauteil einen ersten Halter aufweist, der an der ersten Dichtung befestigt ist und von dieser nach außen absteht sowie eingerichtet ist, bei einem Einführen des Einsetzbauteils in die Aufnahme an den ersten Anschlag anzustoßen und bei einem weiteren Einführen des Einsetzbauteils in die Aufnahme die erste Dichtung zu entfernen und somit das Innere des Einsetzbauteils freizulegen.

Mittels des ersten Halters ist es möglich, die erste Dichtung während des weiteren Einfahrens entlang ihrer gesamten Breite von dem Einsetzbauteil abzuziehen. Dadurch kann ein Kontakt der Intraokularlinse mit der ersten Dichtung in einem Betrieb der Injektoranordnung, in dem die Intraokularlinse mittels eines Kolbens des Injektors verschoben wird, vermieden werden, wodurch ein Risiko einer Beschädigung der Intraokularlinse gering ist. Zudem ist es nicht erforderlich, die erste Dichtung mit einer Hand anzufassen, um die erste Dichtung zu entfernen. Zudem hat die Injektoranordnung einen einfachen Aufbau.

Es ist denkbar, dass der Injektor und das Einsetzbauteil in unterschiedlichen Verpackungen geliefert werden. Hier ist es erforderlich, dass das Einsetzbauteil und der Injektor zuerst von einem Benutzer zusammengefügt werden und dann das Einsetzbauteil in die Aufnahme eingeführt wird, insbesondere durch ein Schieben. Alternativ ist denkbar, dass die Injektoranordnung mit dem an dem Injektor befestigten Einsetzbauteil geliefert wird. Hier ist denkbar, dass in diesem Zustand, in dem das Einsetzbauteil an dem Injektor befestigt ist, das Einsetzbauteil und der Injektor zusammen sterilisiert und dann geliefert werden. So kann eine Kontamination, die erfolgt, während das Einsetzbauteil und der Injektor zusammengefügt werden, wieder beseitigt werden. Von dem Benutzer ist dann lediglich das Einsetzbauteil in die Aufnahme einzuführen, insbesondere durch das Schieben.

Es ist bevorzugt, dass der Injektor einen zweiten Anschlag aufweist und das Einsetzbauteil eine zweite Dichtung aufweist, die außen an dem Einsetzbauteil angeordnet ist und das Innere des Einsetzbauteils abdichtet, wobei das Einsetzbauteil einen zweiten Halter aufweist, der an der zweiten Dichtung befestigt ist und von dieser nach außen absteht sowie eingerichtet ist, bei dem Einführen des Einsetzbauteils in die Aufnahme an den zweiten Anschlag anzustoßen und bei dem weiteren Einführen des Einsetzbauteils in die Aufnahme die zweite Dichtung zu entfernen und somit das Innere des Einsetzbauteils freizulegen.

Es ist bevorzugt, dass die erste Wand ein erstes Durchgangsloch aufweist und die zweite Wand ein zweites Durchgangsloch aufweist, wobei das erste Durchgangsloch fluchtend mit dem zweiten Durchgangsloch angeordnet ist.

Das Einsetzbauteil weist bevorzugt eine erste Einsetzbauteilöffnung, die von der ersten Dichtung abgedichtet ist, und eine zweite Einsetzbauteilöffnung auf, die von der zweiten Dichtung abgedichtet ist, wobei die erste Einsetzbauteilöffnung fluchtend mit der zweiten Einsetzbauteilöffnung angeordnet ist.

Bevorzugt hat das Einsetzbauteil eine Betriebsposition, die das Einsetzbauteil nach dem weiteren Einführen annimmt und in der die beiden Einsetzbauteilöffnungen fluchtend mit den beiden Durchgangslöchern angeordnet sind. Somit kann sich in der Betriebsposition ein Kolben von dem ersten Durchgangsloch via die erste Einsetzbauteilöffnung und die zweite Einsetzbauteilöffnung bis zu dem zweiten Durchgangsloch erstrecken.

Es ist bevorzugt, dass der erste Anschlag von der ersten Wand gebildet ist und/oder der zweite Anschlag von der zweiten Wand gebildet ist.

Es ist bevorzugt, dass der erste Anschlag eine erste Aussparung aufweist, die eingerichtet ist, den ersten Halter aufzunehmen, und/oder wobei der zweite Anschlag eine zweite Aussparung aufweist, die eingerichtet ist, den zweiten Halter aufzunehmen. Mittels der ersten Aussparung kann eine Bewegung des ersten Halters und/oder mittels der zweiten Aussparung kann eine Bewegung des zweiten Halters quer zu einer Einschieberichtung, in der das Einsetzbauteil in die Aufnahme einzuführen ist, bei dem weiteren Einführen des Einsetzbauteils vermieden werden.

Es ist bevorzugt, dass der erste Halter einen Zylinder aufweist, der eingerichtet ist, an dem ersten Anschlag zu drehen, und/oder wobei der zweite Halter einen Zylinder aufweist, der eingerichtet ist, an dem zweiten Anschlag zu drehen. Durch ein geringfügiges Drehen des ersten Halters oder des zweiten Halters während des weiteren Einführens können mechanische Spannungen in der zugehörigen Dichtung vermindert werden. Dadurch sinkt ein Risiko, dass die zugehörige Dichtung während des Einführens reißt.

Die Aufnahme weist bevorzugt eine Nut mit einer Hinterschneidung auf und das Einsetzbauteil weist bevorzugt einen Vorsprung auf, der eingerichtet ist, in die Nut und deren Hinterschneidung einzugreifen. Damit kann sich das Einsetzbauteil lediglich in einer Längsrichtung der Nut bewegen. Die Längsrichtung der Nut ist dabei identisch mit der Einschieberichtung. Es ist besonders bevorzugt, dass die Aufnahme eine dritte Wand aufweist, in der die Nut angeordnet ist und deren nach innen gewandte Oberfläche senkrecht zu der nach innen gewandten Oberfläche der ersten Wand und der nach innen gewandten Oberfläche der zweiten Wand angeordnet ist.

Im Folgenden wird anhand der beigefügten schematischen Zeichnungen die Erfindung näher erläutert.
Figur 1 zeigt eine erfindungsgemäße Injektoranordnung während eines Einfahrens eines Einsetzbauteils,
Figur 2 zeigt die Injektoranordnung aus Figur 1 bei einem weiteren Einfahren des Einsetzbauteils und
Figur 3 zeigt die Injektoranordnung am Ende des Einfahrens des Einsetzbauteils.

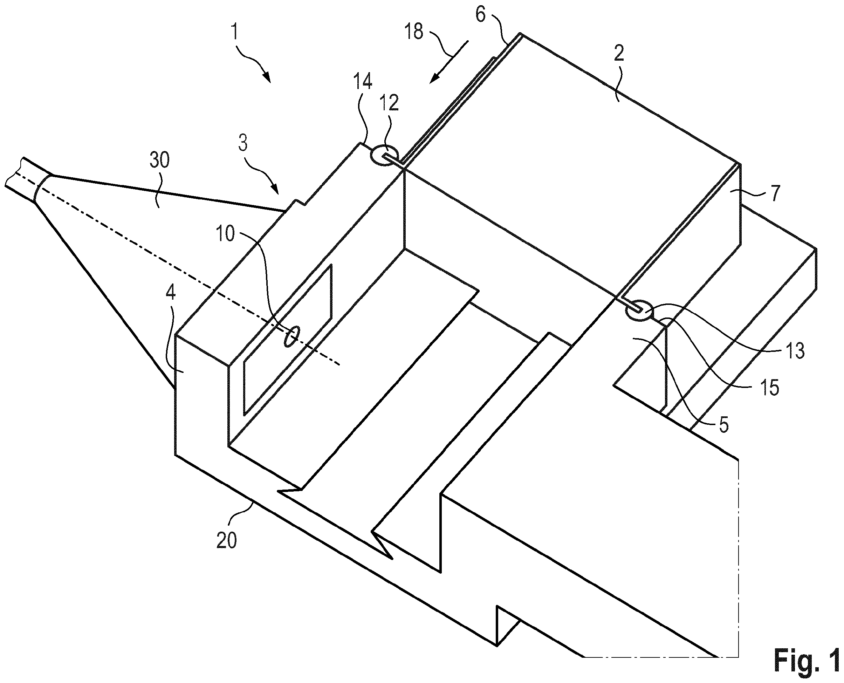

Wie es aus Figuren 1 bis 3 ersichtlich ist, weist eine Injektoranordnung ein Einsetzbauteil 2 und einen Injektor 1 auf. Der Injektor 1 weist einen ersten Anschlag 14 und eine Aufnahme 3 auf. Die Aufnahme 3 weist eine erste Wand 4 und eine zweite Wand 5 auf, wobei die erste Wand 4 und die zweite Wand 5 beabstandet zueinander angeordnet sind. Die Aufnahme 3 ist eingerichtet, zwischen der ersten Wand 4 und der zweiten Wand 5 das Einsetzbauteil 2 gleitend aufzunehmen. Das Einsetzbauteil 2 weist eine Aufbewahrungsflüssigkeit und eine Intraokularlinse auf, die in einem Inneren des Einsetzbauteils 2 umgeben von der Aufbewahrungsflüssigkeit angeordnet ist. Zudem weist das Einsetzbauteil 2 einen ersten Halter 12 und eine erste Dichtung 6 auf, die außen an dem Einsetzbauteil 2 angeordnet ist und das Innere des Einsetzbauteils 2 abdichtet. Der erste Halter 12 ist an der ersten Dichtung 6 befestigt und steht von der ersten Dichtung 6 nach außen ab. Der erste Halter 12 ist eingerichtet, bei einem Einführen des Einsetzbauteils 2 in die Aufnahme 3 an den ersten Anschlag 14 anzustoßen und bei einem weiteren Einführen des Einsetzbauteils 2 in die Aufnahme 3 die erste Dichtung 6 zu entfernen und somit das Innere des Einsetzbauteils 2 freizulegen.

Die erste Wand 4 kann eine nach innen gewandte Oberfläche aufweisen und die zweite Wand 5 kann eine nach innen gewandte Oberfläche aufweisen, wobei die beiden nach innen gewandten Oberflächen einander zugewandt und parallel zueinander angeordnet sind.

Figuren 1 bis 3 zeigen zudem, dass der Injektor 2 einen zweiten Anschlag 15 aufweisen kann und das Einsetzbauteil 2 eine zweite Dichtung 7 aufweisen kann, die außen an dem Einsetzbauteil 2 angeordnet ist und das Innere des Einsetzbauteils 2 abdichtet. Das Einsetzbauteil 2 kann einen zweiten Halter 13 aufweisen, der an der zweiten Dichtung 7 befestigt ist und von der zweiten Dichtung 7 nach außen absteht sowie eingerichtet ist, bei dem Einführen des Einsetzbauteils 2 in die Aufnahme 3 an den zweiten Anschlag 15 anzustoßen und bei dem weiteren Einführen des Einsetzbauteils 2 in die Aufnahme 3 die zweite Dichtung 6 zu entfernen und somit das Innere des Einsetzbauteils 2 freizulegen.

Bei dem weiteren Verschieben des Einsetzbauteils 2 kann ein erster Spalt zwischen der ersten Wand 4 und dem Einsetzbauteil 2 und ein zweiter Spalt zwischen der zweiten Wand 5 und dem Einsetzbauteil 2 vorgesehen sein. Der erste Spalt ist dabei so groß, dass die erste Dichtung 6, während sie entfernt wird, in den ersten Spalt passt, und der zweite Spalt ist dabei so groß, dass die zweite Dichtung 7, während sie entfernt wird, in den zweiten Spalt passt. Dazu können der Abstand von der ersten Wand 4 zu dem von der ersten Dichtung 6 freigelegten Einsetzkörper 2 und der Abstand von der zweiten Wand 5 zu dem von der zweiten Dichtung 7 freigelegten Einsetzkörper 2 jeweils kürzer als 5 mm breit sein, insbesondere kürzer als 3 mm. Der erste Halter 12 und der zweite Halter 13 können dabei entlang der gesamten Breite der zugehörigen Dichtung 6, 7 an dieser befestigt sein. Damit wird während des weiteren Einführens des Einsetzbauteils 2 in die Aufnahme 3 die zugehörige Dichtung 6, 7 auch entlang ihrer gesamten Breite von dem Einsetzkörper 2 entfernt. Dadurch kann es vermieden werden, dass Reste der zugehörigen Dichtung 6, 7 an dem Einsetzkörper 2 verbleiben, die die Intraokularlinse beschädigen können.

Die erste Dichtung 6 und/oder die zweite Dichtung 7 können beispielsweise einen Metallfilm, der von dem Inneren des Einsetzkörper 2 abgewandt angeordnet ist, und einen Kunststofffilm aufweisen, der dem Inneren des Einsetzkörpers 2 zugewandt angeordnet ist und das Innere des Einsetzbauteils 2 abdichtet. Der Metallfilm ist für die mechanische Stabilität der jeweiligen Dichtung 6, 7 zuständig und sorgt insbesondere dafür, dass die jeweilige Dichtung 6, 7 bei ihrem Entfernen nicht reißt, wohingegen der Kunststofffilm für die Dichteigenschaften der jeweiligen Dichtung 6, 7 zuständig ist. Der Einsetzkörper 2 kann durch eine Härtung des Kunststofffilms von diesem abgedichtet werden. Die Härtung kann beispielsweise mittels einer Zufuhr von Wärme erfolgen. Alternativ ist denkbar, dass die erste Dichtung 6 und/oder die zweite Dichtung 7 eine Kunststoffdichtung sind, bevorzugt eine gehärtete Kunststoffdichtung sind.

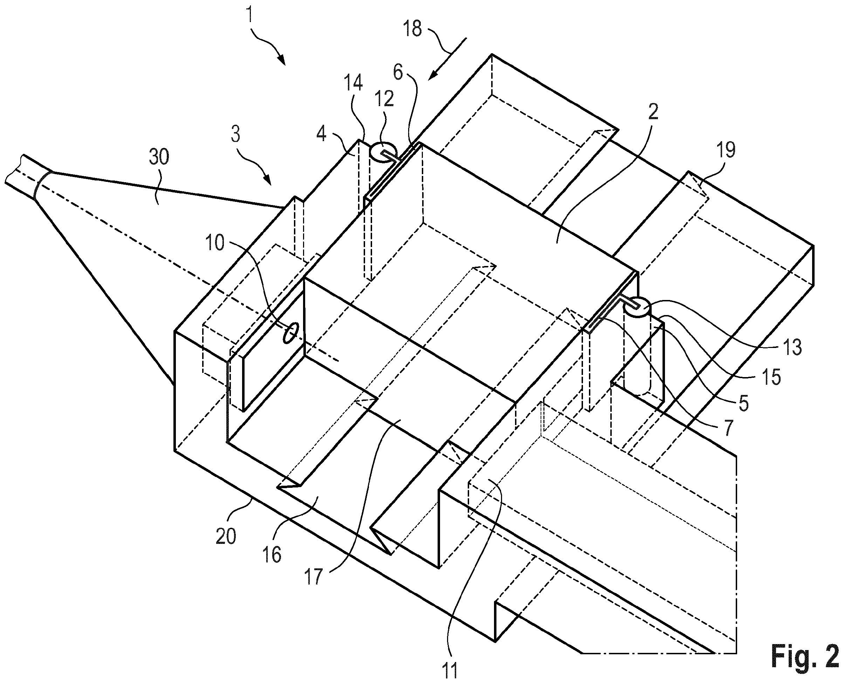

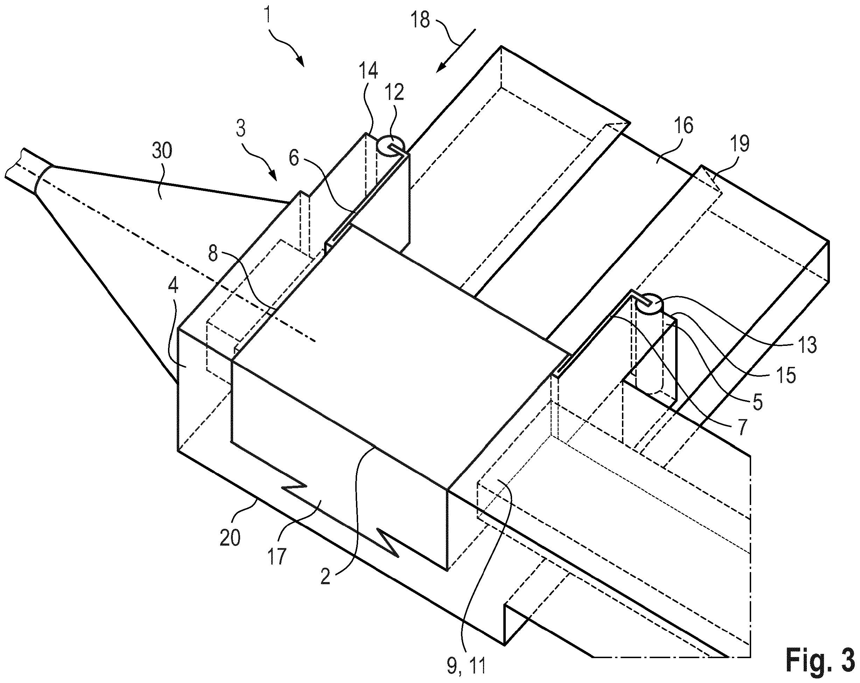

Figur 1 zeigt einen Zustand der Injektoranordnung zu einem ersten Zeitpunkt, an dem der erste Halter 12 an den ersten Anschlag 14 und der zweite Halter 13 an den zweiten Anschlag 15 anstößt. Dabei ist es denkbar, dass die Injektoranordnung in diesem Zustand an einen Benutzer geliefert wird. Es ist auch denkbar, dass der erste Halter 12 einen Abstand von dem ersten Anschlag 14 hat und der zweite Halter 13 einen Abstand von dem zweiten Anschlag 15 hat, wenn die Injektoranordnung geliefert wird. Um die Injektoranordnung gebrauchsfertig zu machen, ist der Einsetzkörper 2 in einer Einschieberichtung 18 in die Aufnahme 3 einzuführen, so dass der erste Halter 12 an den ersten Anschlag 14 und der zweite Halter 14 an den zweiten Anschlag 15 anstoßen. Zudem ist der Einsetzkörper 2 weiter in der Einschieberichtung 18 in die Aufnahme 3 einzuführen, bis sich das Einsetzbauteil 2 in einer in Figur 3 dargestellten Betriebsposition befindet, in der das Innere des Einsetzbauteils 2 freigelegt ist. Figur 2 zeigt einen Zustand des Einsetzbauteils 2, der zeitlich zwischen dem Zustand aus Figur 1 und dem Zustand aus Figur 3 liegt.

Die Figuren zeigen, dass die erste Wand 4 ein erstes Durchgangsloch 10 aufweisen kann und die zweite Wand 5 ein zweites Durchgangsloch 11 aufweisen kann, wobei das erste Durchgangsloch 10 fluchtend mit dem zweiten Durchgangsloch 11 angeordnet ist. Zudem kann das Einsetzbauteil 2 eine erste Einsetzbauteilöffnung 8, die von der ersten Dichtung 6 abgedichtet ist, und eine zweite Einsetzbauteilöffnung 9 aufweisen, die von der zweiten Dichtung 7 abgedichtet ist, wobei die erste Einsetzbauteilöffnung 8 fluchtend mit der zweiten Einsetzbauteilöffnung 9 angeordnet ist. Aus Figur 3 ist ersichtlich, dass in der Betriebsposition des Einsetzbauteils 2, die das Einsetzbauteil 2 nach dem weiteren Einführen annimmt, die beiden Einsetzbauteilöffnungen 8, 9 fluchtend mit den beiden Durchgangslöchern 10, 11 angeordnet sind. Dadurch kann ein Kolben des Injektors 1 in der Betriebsposition von dem zweiten Durchgangsloch 11 via die zweite Einsetzbauteilöffnung 9 in das Innere des Einsetzbauteils 2 geschoben werden. Von dort kann der Kolben die Intraokularlinse via die erste Einsetzbauteilöffnung 8 in das erste Durchgangsloch 10 schieben und von dem ersten Durchgangsloch 10 in eine Kanüle 30 des Injektors 1. Von der Kanüle kann die Intraokularlinse dann in einen Kapselsack eines Auges geschoben werden.

Die Einschieberichtung 18 kann, wie in Figuren 1 bis 3 gezeigt, senkrecht zu einer Längsrichtung des Injektors 1 sein, in der auch der Kolben zu verschieben ist. Alternativ ist denkbar, dass der Einsetzkörper 2 bei dem Einführen und/oder dem weiteren Einführen einer Kreisbahn folgt.

Aus Figuren 1 bis 3 ist ersichtlich, dass der erste Anschlag 14 von der ersten Wand 4 gebildet sein kann und der zweite Anschlag 15 von der zweiten Wand 5 gebildet sein kann. Dabei ist denkbar, dass der erste Anschlag 14 von einer ersten Anschlagoberfläche der ersten Wand 4 gebildet ist, die senkrecht zu der nach innen gewandten Oberfläche der ersten Wand 4 ist, und/oder dass der zweite Anschlag 15 von einer zweiten Anschlagoberfläche der zweiten Wand 5 gebildet ist, die senkrecht zu der nach innen gewandten Oberfläche der zweiten Wand 5 ist. Zudem kann der erste Anschlag 14 eine erste Aussparung aufweisen die eingerichtet ist, den ersten Halter 12 aufzunehmen, und/oder der zweite Anschlag 15 kann eine zweite Aussparung aufweisen, die eingerichtet ist, den zweiten Halter 13 aufzunehmen. Dabei ist denkbar, dass die erste Aussparung in die erste Anschlagoberfläche eingebracht ist und/oder dass die zweite Aussparung in die zweite Anschlagoberfläche eingebracht ist. Der erste Halter 12 kann an dem Längsende der ersten Dichtung 6 angeordnet sein, das zuerst in die Aufnahme 3 eingeführt wird. Der zweite Halter 13 kann an dem Längsende der zweiten Dichtung 7 angeordnet sein, das zuerst in die Aufnahme 3 eingeführt wird. Zudem ist denkbar, dass der erste Halter 12 einen Zylinder aufweist, der eingerichtet ist, an dem ersten Anschlag 14 zu drehen, und/oder wobei der zweite Halter 13 einen Zylinder aufweist, der eingerichtet ist, an dem zweiten Anschlag 15 zu drehen. In diesem Fall können die erste Aussparung und/oder die zweite Aussparung einen kreissegmentförmigen Querschnitt haben.

Wie es aus Figuren 1 bis 3 ersichtlich ist, kann die Aufnahme 3 eine Nut 16 mit einer Hinterschneidung 19 aufweisen und das Einsetzbauteil 2 kann einen Vorsprung 17 aufweisen, der eingerichtet ist, in die Nut 16 und deren Hinterschneidung 19 einzugreifen. Bei der Nut 16 kann es sich um eine Schwalbenschwanznut handeln, und bei dem Vorsprung 17 kann es sich um ein Schwalbenschwanzprofil handeln. Es ist denkbar, dass die Aufnahme 3 eine dritte Wand 20 aufweist, in der die Nut 16 angeordnet ist und deren nach innen gewandte Oberfläche senkrecht zu der nach innen gewandten Oberfläche der ersten Wand 4 und der nach innen gewandten Oberfläche der zweiten Wand 5 angeordnet ist.

### Bezugszeichenliste

1 Injektor
2 Einsetzbauteil
3 Aufnahme
4 erste Wand
5 zweite Wand
6 erste Dichtung
7 zweite Dichtung
8 erste Einsetzbauteilöffnung
9 zweite Einsetzbauteilöffnung
10 erstes Durchgangsloch
11 zweites Durchgangsloch
12 erster Halter
13 zweiter Halter
14 erster Anschlag
15 zweiter Anschlag
16 Nut
17 Vorsprung
18 Einschieberichtung
19 Hinterschneidung
20 dritte Wand
30 Kanüle

## Patentansprüche

1. Injektoranordnung mit einem Einsetzbauteil (2) und einem Injektor (1), der einen ersten Anschlag (14) und eine Aufnahme (3) aufweist, die eine erste Wand (4) und eine zweite Wand (5) aufweist, die beabstandet zueinander angeordnet sind, und eingerichtet ist, zwischen der ersten Wand (4) und der zweiten Wand (5) das Einsetzbauteil (2) gleitend aufzunehmen, das eine Aufbewahrungsflüssigkeit und eine Intraokularlinse, die in einem Inneren des Einsetzbauteils (2) umgeben von der Aufbewahrungsflüssigkeit angeordnet ist, sowie eine erste Dichtung (6) aufweist, die außen an dem Einsetzbauteil (2) angeordnet ist und das Innere des Einsetzbauteils (2) abdichtet, **dadurch gekennzeichnet, dass** das Einsetzbauteil (2) einen ersten Halter (12) aufweist, der an der ersten Dichtung (6) befestigt ist und von dieser nach außen absteht sowie eingerichtet ist, bei einem Einführen des Einsetzbauteils (2) in die Aufnahme (3) an den ersten Anschlag (14) anzustoßen und bei einem weiteren Einführen des Einsetzbauteils (2) in die Aufnahme (3) die erste Dichtung (6) zu entfernen und somit das Innere des Einsetzbauteils (2) freizulegen.

2. Injektoranordnung gemäß Anspruch 1, wobei der Injektor (2) einen zweiten Anschlag (15) aufweist und das Einsetzbauteil (2) eine zweite Dichtung (7) aufweist, die außen an dem Einsetzbauteil (2) angeordnet ist und das Innere des Einsetzbauteils (2) abdichtet, wobei das Einsetzbauteil (2) einen zweiten Halter (13) aufweist, der an der zweiten Dichtung (7) befestigt ist und von dieser nach außen absteht sowie eingerichtet ist, bei dem Einführen des Einsetzbauteils (2) in die Aufnahme (3) an den zweiten Anschlag (15) anzustoßen und bei dem weiteren Einführen des Einsetzbauteils (2) in die Aufnahme (3) die zweite Dichtung (6) zu entfernen und somit das Innere des Einsetzbauteils (2) freizulegen.

3. Injektoranordnung gemäß Anspruch 2, wobei die erste Wand (4) ein erstes Durchgangsloch (10) aufweist und die zweite Wand (5) ein zweites Durchgangsloch (11) aufweist, wobei das erste Durchgangsloch (10) fluchtend mit dem zweiten Durchgangsloch (11) angeordnet ist.

4. Injektoranordnung gemäß Anspruch 3, wobei das Einsetzbauteil (2) eine erste Einsetzbauteilöffnung (8), die von der ersten Dichtung (6) abgedichtet ist, und eine zweite Einsetzbauteilöffnung (9) aufweist, die von der zweiten Dichtung (7) abgedichtet ist, wobei die erste Einsetzbauteilöffnung (8) fluchtend mit der zweiten Einsetzbauteilöffnung (9) angeordnet ist.

5. Injektoranordnung gemäß Anspruch 4, wobei das Einsetzbauteil (2) eine Betriebsposition hat, die das Einsetzbauteil (2) nach dem weiteren Einführen annimmt und in der die beiden Einsetzbauteilöffnungen (8, 9) fluchtend mit den beiden Durchgangslöchern (10, 11) angeordnet sind.

6. Injektoranordnung gemäß einem der Ansprüche 1 bis 5, wobei der erste Anschlag (14) von der ersten Wand (4) gebildet ist und/oder der zweite Anschlag (15) von der zweiten Wand (5) gebildet ist.

7. Injektoranordnung gemäß einem der Ansprüche 1 bis 6, wobei der erste Anschlag (14) eine erste Aussparung aufweist, die eingerichtet ist, den ersten Halter (12) aufzunehmen, und/oder wobei der zweite Anschlag (15) eine zweite Aussparung aufweist, die eingerichtet ist, den zweiten Halter (13) aufzunehmen.

8. Injektoranordnung gemäß einem der Ansprüche 1 bis 7, wobei der erste Halter (12) einen Zylinder aufweist, der eingerichtet ist, an dem ersten Anschlag (14) zu drehen, und/oder wobei der zweite Halter (13) einen Zylinder aufweist, der eingerichtet ist, an dem zweiten Anschlag (15) zu drehen.

9. Injektoranordnung gemäß einem der Ansprüche 1 bis 8, wobei die Aufnahme (3) eine Nut (16) mit einer Hinterschneidung (19) aufweist und das Einsetzbauteil (2) einen Vorsprung (17) aufweist, der eingerichtet ist, in die Nut (16) und deren Hinterschneidung (19) einzugreifen.

10. Injektoranordnung gemäß Anspruch 9, wobei die Aufnahme (3) eine dritte Wand (20) aufweist, in der die Nut (16) angeordnet ist und deren nach innen gewandte Oberfläche senkrecht zu der nach innen gewandten Oberfläche der ersten Wand (4) und der nach innen gewandten Oberfläche der zweiten Wand (5) angeordnet ist.

## Claims

1. Injector assembly comprising an insertion component (2) and an injector (1) which has a first stop (14) and a receptacle (3) which has a first wall (4) and a second wall (5) spaced apart from each other and which is configured to slidably receive the insertion component (2) between the first wall (4) and the second wall (5), the insertion component (2) having a preserving liquid and an intraocular lens which is arranged in an interior of the insertion component (2) and is surrounded by the preserving liquid, and a first seal (6) which is provided on the outside of the insertion component (2) and seals off the interior of the insertion component (2), **characterized in that** the insertion component (2) has a first holder (12) which is fastened to the first seal (6) and protrudes outwards from the latter and which is configured to abut the first stop (14) when the insertion component (2) is introduced into the receptacle (3) and to remove the first seal (6) when the insertion component (2) is introduced further into the receptacle (3), thereby exposing the interior of the insertion component (2).

2. Injector assembly according to Claim 1, wherein the injector (2) has a second stop (15) and the insertion component (2) has a second seal (7) which is arranged on the outside of the insertion component (2) and seals off the interior of the insertion component (2), wherein the insertion component (2) has a second holder (13) which is fastened to the second seal (7) and protrudes outwards from the latter and is configured to abut the second stop (15) when the insertion component (2) is introduced into the receptacle (3) and to remove the second seal (6) when the insertion component (2) is introduced further into the receptacle (3), thereby exposing the interior of the insertion component (2).

3. Injector assembly according to Claim 2, wherein the first wall (4) has a first through-hole (10) and the second wall (5) has a second through-hole (11), wherein the first through-hole (10) is arranged in alignment with the second through-hole (11).

4. Injector assembly according to Claim 3, wherein the insertion component (2) has a first insertion component opening (8), which is sealed by the first seal (6), and a second insertion component opening (9), which is sealed by the second seal (7), wherein the first insertion component opening (8) is arranged in alignment with the second insertion component opening (9).

5. Inj ector assembly according to Claim 4, wherein the insertion component (2) has an operating position which the insertion component (2) assumes after the further introduction and in which the two insertion component openings (8, 9) are arranged in alignment with the two through-holes (10, 11).

6. Injector assembly according to one of Claims 1 to 5, wherein the first stop (14) is formed by the first wall (4), and/or the second stop (15) is formed by the second wall (5).

7. Injector assembly according to one of Claims 1 to 6, wherein the first stop (14) has a first recess, which is configured to receive the first holder (12), and/or wherein the second stop (15) has a second recess, which is configured to receive the second holder (13).

8. Injector assembly according to one of Claims 1 to 7, wherein the first holder (12) has a cylinder which is configured to rotate on the first stop (14), and/or wherein the second holder (13) has a cylinder which is configured to rotate on the second stop (15).

9. Injector assembly according to one of Claims 1 to 8, wherein the receptacle (3) has a groove (16) with an undercut (19), and the insertion component (2) has a projection (17) which is configured to engage in the groove (16) and the undercut (19) of the latter.

10. Injector assembly according to Claim 9, wherein the receptacle (3) has a third wall (20), in which the groove (16) is arranged and of which the inwardly facing surface is perpendicular to the inwardly facing surface of the first wall (4) and the inwardly facing surface of the second wall (5).

## Revendications

1. Ensemble formant injecteur comprenant un composant d'insertion (2) et un injecteur (1) qui comporte une première butée (14) et un logement (3) pourvu d'une première paroi (4) et d'une deuxième paroi (5), disposées à distance l'une de l'autre, et conçu pour recevoir de manière coulissante entre la première paroi (4) et la deuxième paroi (5) l'élément d'insertion (2) qui comporte un liquide de stockage et une lentille intraoculaire disposée dans l'espace intérieur de l'élément d'insertion (2), entouré par le liquide de stockage, et une première garniture d'étanchéité (6) qui est disposée à l'extérieur du composant d'insertion (2) et qui réalise l'étanchéité de l'espace intérieur du composant d'insertion (2), **caractérisé en ce que** le composant d'insertion (2) comporte un premier support (12) qui est fixé à la première garniture d'étanchéité (6) et qui fait saillie de celle-ci vers l'extérieur et est conçue pour venir en butée contre la première butée (14) lorsque le composant d'insertion (2) est inséré dans le logement (3) et pour retirer la première garniture d'étanchéité (6) lorsque le composant d'insertion (2) est davantage inséré dans le logement (3) et ainsi pour libérer l'espace intérieur du composant d'insertion (2).

2. Ensemble formant injecteur selon la revendication 1, l'injecteur (2) comportant une deuxième butée (15) et le composant d'insertion (2) comportant une deuxième garniture d'étanchéité (7) qui est disposée à l'extérieur du composant d'insertion (2) et qui réalise l'étanchéité de l'espace intérieur du composant d'insertion (2), le composant d'insertion (2) comportant un deuxième support (13) qui est fixé à la deuxième garniture d'étanchéité (7) et qui fait saillie de celle-ci vers l'extérieur et qui est conçu pour venir en butée contre la deuxième butée (15) lorsque le composant d'insertion (2) est inséré dans le logement (3) et pour retirer la deuxième garniture d'étanchéité (6) lorsque le composant d'insertion (2) est davantage inséré dans le logement (3) et ainsi pour libérer l'espace intérieur du composant d'insertion (2).

3. Ensemble formant injecteur selon la revendication 2, la première paroi (4) comportant un premier trou traversant (10) et la deuxième paroi (5) comportant un deuxième trou traversant (11), le premier trou traversant (10) étant ménagé en alignement avec le deuxième trou traversant (11).

4. Ensemble formant injecteur selon la revendication 3, le composant d'insertion (2) comportant une première ouverture de composant d'insertion (8) qui est rendue étanche par la première garniture d'étanchéité (6) et une deuxième ouverture de composant d'insertion (9) qui est rendue étanche par la deuxième garniture d'étanchéité (7), ladite première ouverture de composant d'insertion (8) étant alignée avec ladite deuxième ouverture de composant d'insertion (9).

5. Ensemble formant injecteur selon la revendication 4, le composant d'insertion (2) présentant une position de fonctionnement que le composant d'insertion (2) adopte après avoir été davantage inséré et dans laquelle les deux ouvertures de composant d'insertion (8, 9) sont disposées en alignement avec les deux trous traversants (10, 11).

6. Ensemble formant injecteur selon l'une des revendications 1 à 5, la première butée (14) étant formée par la première paroi (4) et/ou la deuxième butée (15) étant formée par la deuxième paroi (5).

7. Ensemble formant injecteur selon l'une des revendications 1 à 6, la première butée (14) comportant un premier évidement qui est conçu pour recevoir le premier support (12), et/ou la deuxième butée (15) comportant un deuxième évidement qui est conçu pour recevoir le deuxième support (13).

8. Ensemble formant injecteur selon l'une des revendications 1 à 7, le premier support (12) comportant un cylindre qui est conçu pour tourner sur la première butée (14), et/ou le deuxième support (13) comportant un cylindre qui est conçu pour tourner sur la deuxième butée (15).

9. Ensemble formant injecteur selon l'une des revendications 1 à 8, le logement (3) comportant une rainure (16) pourvue d'une contre-dépouille (19) et le composant d'insertion (2) comportant une saillie (17) qui est conçue pour s'engager dans la rainure (16) et la contre-dépouille (19) de celle-ci.

10. Ensemble formant injecteur selon la revendication 9, le logement (3) comportant une troisième paroi (20) dans laquelle la rainure (16) est ménagée et la surface de celle-ci dirigée vers l'intérieur étant disposée perpendiculairement à la surface, dirigée vers l'intérieur, de la première paroi (4) et à la surface, dirigée vers l'intérieur, de la deuxième paroi (5).
